# EUROPEAN PATENT APPLICATION

(11) **EP 3 905 259 A1**
(43) Date of publication of application: **03.11.2021**
(21) Application number: 20171662.8
(22) Date of filing: 27.04.2020
(51) Int. Cl.: G16H 50/30, G16H 50/50

(54) **PREDICTIONS FOR CLINICAL DECISION SUPPORT USING PATIENT SPECIFIC PHYSIOLOGICAL MODELS**

(71) Applicant: Siemens Healthcare GmbH, 91052 Erlangen (DE)
(72) Inventor: SHARMA, Puneet, Princeton Junction, NJ New Jersey 08550 (US); NEUMANN, Dominik, 91052 Erlangen (DE); PASSERINI, Tiziano, Plainsboro, NJ 08536 (US)
(74) Representative: EIP

(57) **Abstract**

Systems and methods for determining predictions for clinical decision support using patient specific physiological models are provided. Patient data of a patient is received and physiologically integrated data of the patient is determined using one or more patient specific physiological models. The one or more patient specific physiological models are personalized using the patient data. A prediction for clinical decision making for the patient is determined based on the physiologically integrated data and the patient data using a machine learning network.

## Description

### TECHNICAL FIELD

The present invention relates generally to predictions for clinical decision support using patient specific physiological models, and in particular to predictions for clinical decision support based on physiologically integrated data determined using patient specific physiological models.

### BACKGROUND

Clinical decision support systems are tools that assist clinicians in making clinical decisions. Such clinical decision support systems analyze data to provide information that is helpful for making decisions for diagnosing or treating a patient, thereby improving quality of care, avoiding adverse events, increasing efficiency, and reducing costs. Conventional clinical decision support systems are implemented using predefined rules, statistical models, or machine learning based models. However, such conventional clinical decision support systems typically rely solely on patient data. Accordingly, the accuracy of such conventional clinical decision support systems is limited.

### BRIEF SUMMARY OF THE INVENTION

In accordance with one or more embodiments, predictions for clinical decision support for a patient are determined using patient data of the patient and physiologically integrated data of the patient. The physiologically integrated data is computed using patient specific physiological models personalized using the patient data.

In one embodiment, systems and methods for determining predictions for clinical decision support using patient specific physiological models are provided. Patient data of a patient is received and physiologically integrated data of the patient is determined using one or more patient specific physiological models. The one or more patient specific models are personalized using the patient data. A prediction for clinical decision making for the patient is determined based on the physiologically integrated data and the patient data using a machine learning based system.

In one embodiment, the prediction for clinical decision making is a prediction of a clinical event for the patient, a recommended course of action for the patient, or a risk score associated with a clinical event for the patient. The patient may be classified based on the risk score.

In one embodiment, the physiologically integrated data is determined using a plurality of patient specific physiological models, each modelling a particular physiological function of the patient.

In one embodiment, determining a prediction for clinical decision making includes predicting a plurality of clinical scenarios for the patient. A most likely clinical scenario for the patient may be identified from the plurality of medical conditions, a clinical test for the patient may be recommended to differentiate between the plurality of clinical scenarios, or risks and benefits associated with each of the plurality of clinical scenarios may be identified.

These and other advantages of the invention will be apparent to those of ordinary skill in the art by reference to the following detailed description and the accompanying drawings.

### BRIEF DESCRIPTION OF THE DRAWINGS

Figure 1 shows framework for determining predictions for clinical decision support for a patient, in accordance with one or more embodiments;
Figure 2 shows a workflow for determining predictions for clinical decision support for a patient, in accordance with one or more embodiments;
Figure 3 shows a method for determining predictions for clinical decision support for a patient, in accordance with one or more embodiments; and
Figure 4 shows a high-level block diagram of a computer.

### DETAILED DESCRIPTION

The present invention generally relates to methods and systems for predictions for clinical decision support using patient specific physiological models. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system.

Embodiments described herein provide for predictions for clinical decision support for a patient using both patient data of the patient and physiologically integrated data of the patient. Such physiologically integrated data is computed using patient specific physiological models personalized using the patient data. Advantageously, such physiologically integrated data is computed by considering biophysical laws, extensive clinical and physiological knowledge, and other physiological considerations that are not accounted for in the patient data. Accordingly, embodiments described herein utilize a combination of the patient data and the physiologically integrated data to thereby enhance predictive performance for clinical decision support.

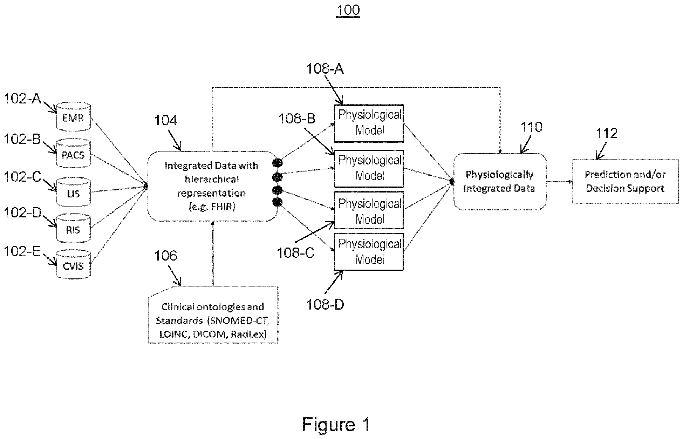

Figure 1 shows a framework 100 for determining predictions for clinical decision support for a patient, in accordance with one or more embodiments. As shown in framework 100, patient data is stored in various disparate data management systems, such as, e.g., EMR (electronic medical records) 102-A, PACS (picture archiving and communication system) 102-B, LIS (laboratory information system) 102-C, RIS (radiology information system) 102-D, and CVIS (cardiovascular information system) 102-E (collectively referred to as data management systems 102). The patient data stored in the various data management systems 102 is represented in accordance with various clinical ontologies and standards 106 (e.g., SNOMED-CT (systematized nomenclature of medicine - clinical terms), LOINC (logical observation identifiers names and codes), ICD (international classification of diseases) codes, etc.), and combined as integrated data 104 with hierarchical representation. Integrated data 104 may be integrated according to the FHIR (fast healthcare interoperability resources) specification or any other standard for data interoperability. Integrated data 104 is input into patient specific physiological models 108-A, 108-B, 108-C, and 108-D (collectively referred to as patient specific physiological models 108), which output physiologically integrated data 110. Each patient specific physiological model 108 models a different physiological function, such as, e.g., the function of a particular organ under different patient states, the manifestation and progression of a disease, the interaction between medical devices or drugs and organs, the interaction between multiple organs, the physiological response of treatments, etc. The physiologically integrated data 110 and the integrated data 104 are input into a machine learning based system for prediction and/or decision support 112.

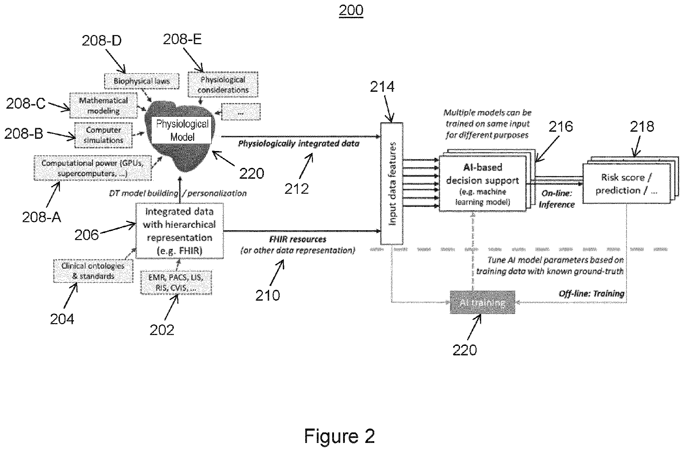

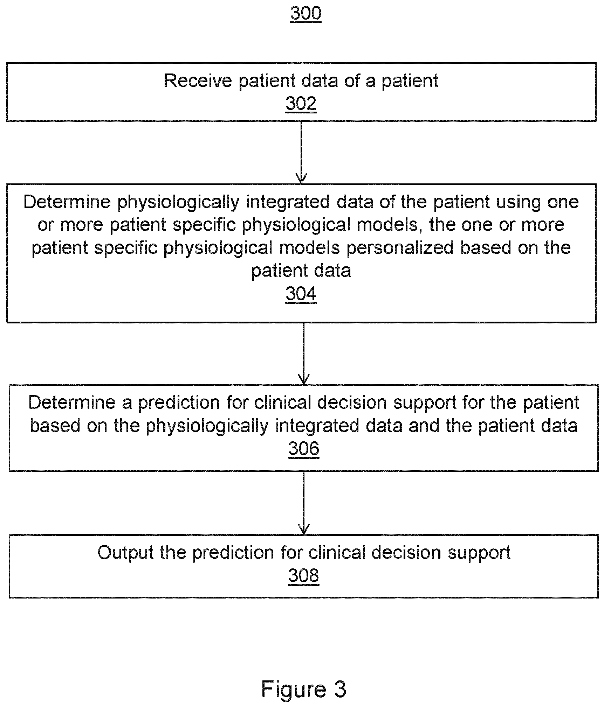

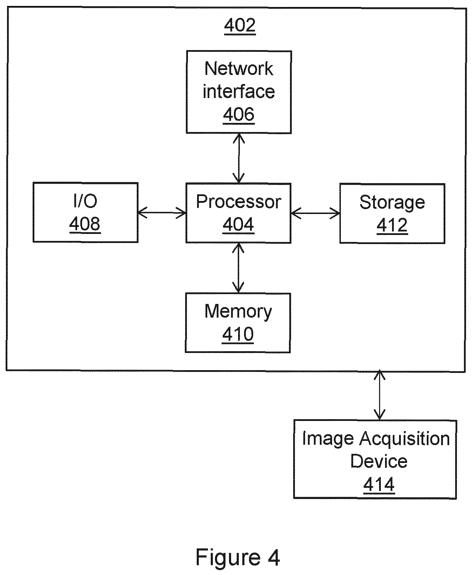

Figure 2 shows a workflow 200 for determining predictions for clinical decision support for a patient, in accordance with one or more embodiments. Figure 3 shows a method 300 for determining predictions for clinical decision support for a patient, in accordance with one or more embodiments. Figures 2 and 3 will be simultaneously described with continued reference to Figure 1. The steps of method 300 may be performed by one or more suitable computing devices, such as, e.g., computed 402 of Figure 4.

At step 302, patient data of a patient is received. In one example, the patient data is integrated data 104 of Figure 1, which is a combination of data from EMR 102-A, PACS 102-B, LIS 102-C, RIS 102-D, and CVIS 102-E in accordance with clinical ontologies and standards 106. In another example, the patient data is integrated data 206 of Figure 2, which is a combination of data from EMR, PACS, LIS, RIS, CVIS, etc. 202 in accordance with clinical ontologies and standards 204. The patient data may include any data relating to the patient, such as, e.g., medical records, medical imaging data, laboratory data, drug and prescription data, demographic information, etc. In one embodiment, the patient data is hierarchically represented according to the FHIR specification or any other interoperability standard.

At step 304, physiologically integrated data of the patient is determined using one or more patient specific physiological models. The patient specific physiological models are personalized based on the patient data. In one example, the physiologically integrated data is physiologically integrated data 110 determined using patient specific physiological models 108 in Figure 1. In another example, the physiologically integrated data is physiologically integrated data 212 determined from patient specific physiological model 220 in Figure 2. Patient specific physiological model 220 is exemplarily shown as a virtual heart modelling the physiological function of a heart in Figure 2.

A patient specific physiological model is a computational model of a particular physiological function for a particular patient. The patient specific physiological model may model a physiological function using various resources. For example, as shown in Figure 2, patient specific physiological model 220 models physiological function of a heart using computational power 208-A (e.g., GPUs (graphics processing units), supercomputers, etc.), computer simulations 208-B, mathematical modeling 208-C, biophysical laws 208-D, physiological considerations 208-E, or any other suitable physiological factor. Patient specific physiological model 220 is personalized for the patient based on integrated data 206.

The patient specific physiological model may model any physiological function of the patient. For example, the patient specific physiological model may be a physiological model of a particular anatomical structure of the patient under various conditions. An example of anatomical structures that may be modeled by the patient specific physiological model is an organ, such as, e.g., the heart, lungs, liver, brain, arterial circulation, and musculoskeletal structures. Conditions may include a baseline condition (e.g., a rest state of the patient, a non-drug-induced state of the patient, etc.) and a non-baseline condition (e.g., an exercise state, a drug induced state, etc.). Other examples of the patient specific physiological model include a computational model of disease manifestation and progression, a computation model of the interaction between medical devices and/or drugs and a particular organ, a computational model of the interaction between a number of different organs, a computational model of a virtual (i.e., simulated) intervention or treatment. In some embodiments, a plurality of patient specific physiological models are utilized each modeling a particular physiological function.

The patient specific physiological model is personalized based on the patient data. In one embodiment, the patient specific physiological model is constructed and personalized as described in U.S. Patent Publication No. 2016/0210435, filed February 29, 2016, U.S. Patent No. 10,496,729, filed February 24, 2015, or U.S. Patent Publication No. 2016/0196384, filed December 17, 2015, the disclosures of which are incorporated herein by reference in their entirety.

The physiologically integrated data includes outputs from the patient specific physiological models as well as parameters of the patient specific physiological models. The personalization of the patient specific physiological models makes the parameters potentially relevant for decision making. Personalized parameters 1) make the outputs of the patient specific physiological models consistent with the patient data and 2) assume values that are specific for the patient, while having a physiological meaning because of the underlying patient specific physiological models.

At step 306, a prediction for clinical decision support for the patient is determined based on the physiologically integrated data and the patient data. In one example, the prediction for clinical decision support is prediction and/or decision support 112 of Figure 1 or risk score/prediction 218 of Figure 2.

The prediction for clinical decision support includes any prediction for supporting clinical decision making for diagnosing or treating the patient. In one example, the prediction for clinical decision support is a prediction of a clinical event, such as, e.g., adverse or non-adverse events, outcomes of a therapy, course of disease evolution, or any other medical event. In another example, the prediction for clinical decision support is a recommended course of action, such as, e.g., recommended treatments, recommended diagnostic or therapeutic tests, etc. The predicted clinical events and/or the recommended course of action may be ranked based on, e.g., likelihood of occurrence, likelihood of success, or any other factor. In another example, the prediction for clinical decision support includes a predicted risk (e.g., a risk score) associated with a clinical event or recommended course of action, such as, e.g., adverse or non-adverse events, performing an intervention (or not performing an intervention), readmission after performing a medical procedure, recurrence of a disease or condition, etc. The patient may be classified into risk categories or grounds based on the predicted risk using one or more thresholds. In another example, the prediction for clinical decision support includes a priority of the patient among a group of patients based on, e.g., an urgency or risk category. Other predictions for clinical decision support are also contemplated.

In one embodiment, the prediction for clinical decision support is determined using a trained machine learning based system based on the physiologically integrated data and the patient data. For example, as shown in Figure 2, physiologically integrated data 212 (e.g., model outputs and parameters) and integrated data 206 represented as FHIR resources 210 are combined into input data features 214 and input into AI (artificial intelligence) based decision support 216 to determine risk score/prediction 218. The machine learning based system may be a machine learning model, such as, e.g., a regression or classification model. The machine learning based system is trained during a prior offline training stage using AI training data 220 comprising training input data (patient data and physiologically integrated data) with known ground truth values to tune parameters of the machine learning based system. During the training stage, the machine learning based system extracts relevant features from the training input data. Once trained, the machine learning based system is applied during an online inference stage to determine risk score/prediction 218. Specialized models may be designed, for which specific combinations of patient data and physiologically integrated data can be used to define the input data for the machine learning based system. In one embodiment, the machine learning based system comprises a plurality of machine learning models trained to determine different predictions for clinical decision support using the same input.

At step 308, the prediction for clinical decision support is output. For example, the prediction for clinical decision support can be output by displaying the prediction for clinical decision support on a display device of a computer system, audibly communicating the prediction for clinical decision support via a voice/dialog system, storing the prediction for clinical decision support on a memory or storage of a computer system, or by transmitting the prediction for clinical decision support to a remote computer system.

Advantageously, embodiments described herein utilize both patient data and physiologically integrated data to determine predictions for clinical decision support, thereby combining a data-driven approach and a physiological model-based approach for improved prediction performance.

In one embodiment, the physiologically integrated data may be transmitted to, and stored by, data management systems from which the patient data was retrieved. For example, the data management systems may include data management system 102 of Figure 1 or EMR, PACS, LIS, RIS, CVIS 202 of Figure 2. The physiologically integrated data may be transmitted to such data management systems by extending the FHIR model with additional resources.

In one embodiment, the patient specific physiological models may be used to curate the patient data. Patient data, such as, e.g., measurements and medical reports, may be conflicting or inconsistent due to measurement error or the nature of the data itself. For example, blood test results may be inaccurate where the patient did not properly fast, where the blood test was performed during an ongoing unreported clinical condition of the patient (e.g., viral infection), or where the blood was contaminated by bacteria on the skin. The patient specific physiological models may detect abnormalities or inconsistencies in the patient data and transmit a notification (e.g., a warning) or provide a recommendation (e.g., a retest). In another example, a patient specific physiological model of an immune system may compute the expected dynamics of white blood cells based on patient history, risk factors, and past and present blood exams. Dynamic changes in white blood cell count may be used to identify blood tests where bacterial contamination is found but significant changes in the white blood cell count is absent. Other patient specific physiological models may also be applied to identify inconsistencies in patient data, such as, e.g., a patient specific physiological model of the pancreas to evaluate potential comorbidities (or complications thereof) such as diabetes mellitus.

In one embodiment, the patient specific physiological models may be utilized to determine a more accurate differential diagnosis and recommend additional tests to rule out various conditions. In particular, the machine learning based system utilizes the physiologically integrated data determined using the patient specific physiological models to predict different clinical scenarios (e.g., the progression of medical conditions, treatments, or interventions) of the patient in order to differentiate between the medical conditions. The machine learning based system may output a most likely clinical scenario according to the patient specific physiological models, or request additional information (e.g., recommend clinical tests for the patient) to address inconsistency detected in the data or to differentiate between the clinical scenarios of the patient. Applying multiple patient specific physiological models on available patient data allows for the early identification of interactions between different organs, systems, and treatment options, enabling a more comprehensive and informed handling of a clinical case.

In one embodiment, physiological models may be selected or tailored to according to specific populations, characteristics, or conditions before being personalized using the patient data. For example, physiological models may be selected based on a particular demographic or geographic location, such as, e.g., a pediatric or elderly population or a male or female population. Model selection and tailoring may be performed automatically based on the patient data or by the user where prior knowledge regarding the observed population is available. In one example, normal ranges for hematocrit and hemoglobin in residents of high altitude geographic locations are known to be significantly higher than in the general population, suggesting an adapted physiology. Accordingly, a physiological model may be tailored to account for known physiological adaptions, e.g., resulting from environmental conditions.

In one use case, embodiments described herein may be applied to determine a prediction for clinical decision support for a patient with severe aortic valve stenosis, concomitant coronary artery disease (CAD), and chronic kidney disease. Physiologically integrated data may be determined using patient specific physiological models for modeling valve function (including hemodynamics, leaflet motion, etc.), artificial valve function, coronary circulation, renal function (including the modeling of disease generation and progression), and arterial circulation. The prediction for clinical decision support may answer the clinical question of interest of "what is the best treatment option for this patient," considering that severe aortic valve stenosis is associated with significant mortality, CAD patients undergoing aortic valve replacement have a document increased risk of death, myocardial infarction, and stroke, aortic stenosis worsens outcome for chronic kidney disease patients, and valve replacement is associated with improved survival and renal function. Conventional rule-based decision making is subject to conflicting recommendations and is not suitable in this case. However, in accordance with embodiments described herein, the risk associated with the planned procedure and various scenarios can be predicted. For instance, one cause of concern in the management of aortic stenosis patients with CAD is the potential effect of the valve replacement procedure in causing ischemia or hemodynamic instability. The manifestation of sustained hypotension or hemodynamic collapse during valve replacement is a major risk factor that is correlated with a poor outcome for the valve replacement procedure. A patient specific physiological model of the coronary circulation may be utilized to clarify adverse effects of the valve replacement procedure, for example, relating to the size or target position of a valve replacement device, before performing the treatment. Accordingly, the patient specific physiological model enables improved procedural planning to switch from a transcutaneous to a surgical valve replacement approach, during which hemodynamics instability could also be directly handled. Further, patient specific physiological models may be utilized to predict the risk or benefit of undergoing percutaneous coronary intervention (PCI) or coronary artery bypass grafting (CABG) before the valve replacement procedure to stabilize hemodynamics. Significant risks in this case comes from the adverse effects of postponing the valve replacement procedure. By virtually simulating the scenarios using patient specific physiological models, benefits and disadvantages can be weighed to select the best therapeutic approach without postponing the valve replacement procedure.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatus, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatus, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figure 3. Certain steps or functions of the methods and workflows described herein, including one or more of the steps or functions of Figure 3, may be performed by a server or by another processor in a network-based cloud-computing system. Certain steps or functions of the methods and workflows described herein, including one or more of the steps of Figure 3, may be performed by a client computer in a network-based cloud computing system. The steps or functions of the methods and workflows described herein, including one or more of the steps of Figure 3, may be performed by a server and/or by a client computer in a network-based cloud computing system, in any combination.

Systems, apparatus, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figure 3, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 402 that may be used to implement systems, apparatus, and methods described herein is depicted in Figure 4. Computer 402 includes a processor 404 operatively coupled to a data storage device 412 and a memory 410. Processor 404 controls the overall operation of computer 402 by executing computer program instructions that define such operations. The computer program instructions may be stored in data storage device 412, or other computer readable medium, and loaded into memory 410 when execution of the computer program instructions is desired. Thus, the method and workflow steps or functions of Figures 2-4 can be defined by the computer program instructions stored in memory 410 and/or data storage device 412 and controlled by processor 404 executing the computer program instructions. For example, the computer program instructions can be implemented as computer executable code programmed by one skilled in the art to perform the method and workflow steps or functions of Figures 2-4. Accordingly, by executing the computer program instructions, the processor 404 executes the method and workflow steps or functions of Figures 2-4. Computer 402 may also include one or more network interfaces 406 for communicating with other devices via a network. Computer 402 may also include one or more input/output devices 408 that enable user interaction with computer 402 (e.g., display, keyboard, mouse, speakers, buttons, etc.).

Processor 404 may include both general and special purpose microprocessors, and may be the sole processor or one of multiple processors of computer 402. Processor 404 may include one or more central processing units (CPUs), for example. Processor 404, data storage device 412, and/or memory 410 may include, be supplemented by, or incorporated in, one or more application-specific integrated circuits (ASICs) and/or one or more field programmable gate arrays (FPGAs).

Data storage device 412 and memory 410 each include a tangible non-transitory computer readable storage medium. Data storage device 412, and memory 410, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 408 may include peripherals, such as a printer, scanner, display screen, etc. For example, input/output devices 408 may include a display device such as a cathode ray tube (CRT) or liquid crystal display (LCD) monitor for displaying information to the user, a keyboard, and a pointing device such as a mouse or a trackball by which the user can provide input to computer 402.

An image acquisition device 414 can be connected to the computer 402 to input image data (e.g., medical images) to the computer 402. It is possible to implement the image acquisition device 414 and the computer 402 as one device. It is also possible that the image acquisition device 414 and the computer 402 communicate wirelessly through a network. In a possible embodiment, the computer 402 can be located remotely with respect to the image acquisition device 414.

Any or all of the systems and apparatus discussed herein, including data management systems 102 and patient specific physiological models 108 of Figure 1 and Al-based decision support system 216 and patient specific physiological model 220 of Figure 2, may be implemented using one or more computers such as computer 402.

One skilled in the art will recognize that an implementation of an actual computer or computer system may have other structures and may contain other components as well, and that Figure 4 is a high level representation of some of the components of such a computer for illustrative purposes.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope and spirit of the invention. Those skilled in the art could implement various other feature combinations without departing from the scope and spirit of the invention.

## Claims

1. A method comprising:
receiving (302) patient data of a patient;
determining (304) physiologically integrated data of the patient using one or more patient specific physiological models, the one or more patient specific physiological models personalized using the patient data; and
determining (306) a prediction for clinical decision making for the patient based on the physiologically integrated data and the patient data.

2. The method of claim 1, wherein determining a prediction for clinical decision making for the patient based on the physiologically integrated data and the patient data comprises:
determining a prediction of a clinical event for the patient.

3. The method of claim 1, wherein determining a prediction for clinical decision making for the patient based on the physiologically integrated data and the patient data comprises:
determining a recommended course of action for the patient.

4. The method of claim 1, wherein determining a prediction for clinical decision making for the patient based on the physiologically integrated data and the patient data comprises:
determining a risk score associated with a clinical event for the patient.

5. The method of claim 4, wherein determining a prediction for clinical decision making for the patient based on the physiologically integrated data and the patient data comprises:
classifying the patient based on the risk score.

6. The method of claim 1, wherein determining physiologically integrated data of the patient using one or more patient specific physiological models comprises:
determining the physiologically integrated data of the patient using a plurality of patient specific physiological models, each modelling a particular physiological function of the patient.

7. The method of claim 1, wherein determining a prediction for clinical decision making for the patient based on the physiologically integrated data and the patient data comprises:
determining the prediction using a machine learning based system.

8. The method of claim 1, wherein determining a prediction for clinical decision making for the patient based on the physiologically integrated data and the patient data comprises:
predicting a plurality of clinical scenarios for the patient; and
identifying a most likely clinical scenario for the patient from the plurality of clinical scenarios.

9. The method of claim 1, wherein determining a prediction for clinical decision making for the patient based on the physiologically integrated data and the patient data comprises:
predicting a plurality of clinical scenarios for the patient; and
recommending a clinical test for the patient to differentiate between the plurality of clinical scenarios.

10. The method of claim 1, wherein determining a prediction for clinical decision making for the patient based on the physiologically integrated data and the patient data comprises:
predicting a plurality of clinical scenarios for the patient; and
identifying risks and benefits associated with each of the plurality of clinical scenarios.

11. An apparatus comprising:
means for receiving (302) patient data of a patient;
means for determining (304) physiologically integrated data of the patient using one or more patient specific physiological models, the one or more patient specific physiological models personalized using the patient data; and
means for determining (306) a prediction for clinical decision making for the patient based on the physiologically integrated data and the patient data.

12. The apparatus of claim 11, wherein the means for determining a prediction for clinical decision making for the patient based on the physiologically integrated data and the patient data comprises:
means for determining a prediction of a clinical event for the patient.

13. The apparatus of claim 11, wherein the means for determining a prediction for clinical decision making for the patient based on the physiologically integrated data and the patient data comprises:
means for determining a recommended course of action for the patient.

14. The apparatus of claim 11, wherein the means for determining a prediction for clinical decision making for the patient based on the physiologically integrated data and the patient data comprises:
means for determining a risk score associated with a clinical event for the patient.

15. The apparatus of claim 14, wherein the means for determining a prediction for clinical decision making for the patient based on the physiologically integrated data and the patient data comprises:
means for classifying the patient based on the risk score.

16. A non-transitory computer readable medium storing computer program instructions, the computer program instructions when executed by a processor cause the processor to perform operations comprising:
receiving (302) patient data of a patient;
determining (304) physiologically integrated data of the patient using one or more patient specific physiological models, the one or more patient specific physiological models personalized using the patient data; and
determining (306) a prediction for clinical decision making for the patient based on the physiologically integrated data and the patient data.

17. The non-transitory computer readable medium of claim 16, wherein determining physiologically integrated data of the patient using one or more patient specific physiological models comprises:
determining the physiologically integrated data of the patient using a plurality of patient specific physiological models, each modelling a particular physiological function of the patient.

18. The non-transitory computer readable medium of claim 16, wherein determining a prediction for clinical decision making for the patient based on the physiologically integrated data and the patient data comprises:
predicting a plurality of clinical scenarios for the patient; and
identifying a most likely clinical scenario for the patient from the plurality of clinical scenarios.

19. The non-transitory computer readable medium of claim 16, wherein determining a prediction for clinical decision making for the patient based on the physiologically integrated data and the patient data comprises:
predicting a plurality of clinical scenarios for the patient; and
recommending a clinical test for the patient to differentiate between the plurality of clinical scenarios.

20. The non-transitory computer readable medium of claim 16, wherein determining a prediction for clinical decision making for the patient based on the physiologically integrated data and the patient data comprises:
predicting a plurality of clinical scenarios for the patient; and
identifying risks and benefits associated with each of the plurality of clinical scenarios.
